# EUROPEAN PATENT SPECIFICATION

(11) **EP 2 053 123 B1**
(45) Date of publication and mention of the grant of the patent: **15.01.2014**
(21) Application number: 07791749.0
(22) Date of filing: 01.08.2007
(51) Int. Cl.: A61K 35/14, A61P 31/00, A61P 35/00, A61P 37/02, C12N 5/0783

(54) **METHOD OF PROLIFERATING LAK CELL**
VERFAHREN ZUR PROLIFERATION EINER LAK-ZELLE
PROCÉDÉ DE PROLIFÉRATION D'UNE CELLULE LAK

(30) Priority: 01.08.2006 JP 2006209979; 15.02.2007 JP 2007035134
(43) Date of publication of application: 29.04.2009
(73) Proprietor: NAI Inc., Minato-ku Tokyo 107-0061 (JP)
(72) Inventor: WATARAI, Shinobu, Sakai-shi Osaka 591-8037 (JP); NISHIKAWA, Shigeru, Osaka-shi Osaka 553-0007 (JP)
(74) Representative: Denison, Christopher Marcus
(86) International application number: PCT/JP2007/065068
(87) International publication number: WO 2008/016078

(56) References cited:
- JP-A- 2001 354 575
- JP-A- 2002 171 966
- JP-A- 2002 332 244
- KOMADA H ET AL: "Early calcium signaling and calcium requirements for the IL-2 receptor expression and IL-2 production in stimulated lymphocytes." CELLULAR IMMUNOLOGY 1 NOV 1996 LNKD- PUBMED:8912879, vol. 173, no. 2, 1 November 1996 (1996-11-01), pages 215-220, XP002598443 ISSN: 0008-8749
- KATO MASAHIRO ET AL: "A novel culture method of canine peripheral blood lymphocytes with concanavalin a and recombinant human interleukin-2 for adoptive immunotherapy." THE JOURNAL OF VETERINARY MEDICAL SCIENCE / THE JAPANESE SOCIETY OF VETERINARY SCIENCE MAY 2007 LNKD- PUBMED:17551220, vol. 69, no. 5, May 2007 (2007-05), pages 481-486, XP002598444 ISSN: 0916-7250
- ITOH HIROSHI ET AL: "Bulk cultures of canine peripheral blood lymphocytes with solid phase anti-CD3 antibody and recombinant interleukin-2 for use in immunotherapy." THE JOURNAL OF VETERINARY MEDICAL SCIENCE / THE JAPANESE SOCIETY OF VETERINARY SCIENCE MAR 2003 LNKD- PUBMED:12679562, vol. 65, no. 3, March 2003 (2003-03), pages 329-333, XP002598445 ISSN: 0916-7250
- FENWICK B.W. ET AL.: 'Human recombinant interleukin-2 (125) induced in vitro proliferation of equine, caprine, ovine, canine and feline peripheral blood lymphocytes' COMP. IMMUNOL. MICROBIOL. INFECT. DIS. vol. 11, no. 1, 1988, pages 51 - 60, XP003020875
- TOMIMATSU H. ET AL.: 'Recombinant interleukin 2 ni yoru Kenjojin Oyobi Tangan Kanja Masshoketsu Lymphocyte no Kasseika ni Okeru Kakushu mitogen no Koka' JOURNAL OF TOKYO WOMEN'S MEDICAL UNIVERSITY vol. 65, no. 11, 1995, pages 954 - 962, XP003020876
- SPETZ A.L. ET AL.: 'Induction of CD8 molecules on thymic gamma/delta T cells in vitro is dependent upon alpha/beta T cells' EUR. J. IMMUNOL. vol. 21, no. 11, 1991, pages 2755 - 2759, XP003020877
- GRAFF J.C. ET AL.: 'A comprehensive SAGE database for the analysis of gammadelta T cells' INT. IMMUNOL. vol. 18, no. 4, April 2006, pages 613 - 626, XP003020878
- ITO H. ET AL.: 'Inu ni Okeru Gan Men'eki Ryoho no Rinsho Oyo' DAI 134 KAI JAPANESE SOCIETY OF VETERIANY SCIENCE GAKUJUTSU SHUKAI KOEN YOSHISHU 2002, page 83, XP003020879
- LIU S.Q. ET AL.: 'Controlled growth and release of CD4+T lymphocytes by Concanavalin A-immobilized matrices' PROCEEDINGS OF THE INTERNATIONAL SYMPOSIUM ON CONTROLLED RELEASE OF BIOACTIVE MATERIALS vol. 23, 1996, pages 817 - 818, XP003020880
- KATO M. ET AL.: 'Men'eki Saibo Ryoho eno Oyo ni Muketa Concanavalin A Oyobi Kumikae Hito Interleukin 2 o Mochiita Inu Masshoketsu Lymphocyte no Shinki Baiyoho' DAI 143 KAI JAPANESE SOCIETY OF VETERIANY SCIENCE GAKUJUTSU SHUKAI KOEN YOSHISHU 15 March 2007, page 65 1 ABSTR. NO. PL-5, XP003020881
- MIZUNO S. ET AL.: 'Changes in lymphokine-activated killer activity in peripheral blood lymphocytes from canine transmissible venereal sarcoma models' EXP. ANIM. vol. 45, no. 3, 1996, pages 289 - 292, XP003020882
- ZHAO Y. ET AL.: 'Enhanced expression of novel CD57+CD8+ LAK cells from cats infected with feline immunodeficiency virus' J. LEUKOC. BIOL. vol. 58, no. 4, 1995, pages 423 - 431, XP003020883
- M. OTSU: 'Lack of dominant-negative effects of a truncated gammac on retroviral-mediated gene correction of immunodeficient mice' BLOOD vol. 97, no. 6, 15 March 2001, pages 1618 - 1624, XP055006236 DOI: 10.1182/blood.V97.6.1618 ISSN: 0006-4971

## Description

### TECHNICAL FIELD

This invention relates to a method for proliferating LAK cells, and a cell culture kit for use in proliferating LAK cells.

### BACKGROUND ART

It is generally known in the art that a treatment method, such as a surgical therapy by removing an affected lesion, a chemotherapy by administering anticancer agents, and a radiation therapy by applying radiation to an affected lesion, can be used as a means for treating cancers and/or tumors.

However, these therapies involve some disadvantages for the patients such as a major physical burden and adverse effects associated with chemical agents or ionized radiation. Thus, since 1980's, as a fourth therapy, a therapy using activated autologous lymphocytes, such as LAK (lymphokine-activated killer) therapy and CTL (cytotoxic T lymphocyte) therapy, has been investigated and has become used to treat human cancerous diseases (see, Non-Patent Document 1).

In the art, the term "LAK therapy" refers to a method for treating a cancer of a patient using immunity exerted by autologous lymphocytes of the patient which were proliferated and activated ex vivo. Specifically, "LAK therapy" is a method for treating a cancer by the following steps: (1) collecting blood from a patient who suffers from a cancer, and isolating a fraction containing lymphocytes; (2) proliferating and activating lymphocytes by culturing thus isolated fraction in the culture medium supplemented with interleukin-2 and anti-CD3 antibody for about two weeks; (3) washing thus obtained cell suspension containing a number of lymphocytes, eliminating interleukin-2, anti-CD3 antibody, and the culture medium from the cells, and suspending thus obtained lymphocytes in an appropriate solution (such as a physiological saline for infusion containing an albumin) to prepare a cellular formulation, and (4) administering the cellular formulation to returning the activated lymphocytes back to the patient body. Also, the CTL therapy is a method using a cytotoxic T lymphocytes, in place of LAK cells, after proliferating and activating the cytotoxic T lymphocytes.

A therapy using activated autologous lymphocytes has been improved in various points like other therapy. For example, by culturing a cell suspension containing the autologous lymphocytes in a culture medium supplemented with T cells mitogens (such as concanavalin A) and cytokine (such as interleukin 7), in place of an anti-CD3 antibody, a method for effectively treating a cancer by specifically proliferating/activating γd-type T cells (which directly involve in cell-mediated immunity), rather than activating the entire lymphocytes, has been developed in the art (see, Patent Documents 1 and 2). A method of preparing LAK cells from PBMCs obtained from cats infected with FIV in which the PBMC are first treated with 5 micrograms/ml of ConA for 24h and then washed before being treated with IL-2 at 2 U/ml has also been described (Non-Patent Document 2).

In the art, the therapy using the activated autologous lymphocytes (such as LAK therapy or CTL therapy) has been tried to be applied to diseases other than a cancer (such as immunological diseases or infectious diseases), as described in Patent Documents 1 and 2.

However, there are some problems to be solved such as follows. First, since a CD3 protein used as an immunogen for eliciting an anti-CD3 antibody varies by species originated (such as a dog, a cat, domestic animals, and a human), anti-CD3 antibodies have to be prepared for each species to be treated. Further, the anti-CD3 antibodies, especially an anti-CD3 antibody for a human now generally used in the art, are very expensive. Therefore, the therapy using the activated autologous lymphocytes (such as LAK therapy or CTL therapy) is considered to be hard to be applied to non-human animals from the economical viewpoint.

It is also considered as a problem that, since the γd-type T cells does not demonstrate an antigen specificity or an MHC restriction even through proliferating γd-type T cells, the γd-type T cells are not so useful for the therapy using the activated autologous lymphocytes (such as LAK therapy or CTL therapy).
Patent Document 1: Japanese Patent Domestic Announcement No.2002-528115 (JP-2002-528115-W)
Patent Document 2: Japanese Patent Domestic Announcement No.2003-529363 (JP-2003-529363-W)
Non-Patent Document 1: [searched on June 27, 2006], on the Internet <URL: http://www.j-immunother.com/treatment/02Treatment.html>
Non-Patent Document 2: Zhao et al., 1995, Journal of Leukocyte Biology, 58:423-431

### DISCLOSURE OF THE INVENTION

### PROBLEMS TO BE SOLVED BY THE INVENTION

The present application aims at providing a treatment method effective for various cancers, immunodeficiency diseases and infections, and a method of proliferating/activating cells associated therewith, which can be performed at such low costs that the methods can be applicable to nonhuman animals.

### MEANS FOR SOLVING THE PROBLEMS

The main feature of the present invention is to supplement a plant lectin (such as concanavalin A) and a growth factor having an interleukin-2-like activity to a culture medium, upon culturing cells obtained from a start specimen.

### BRIEF DESCRIPTION OF THE DRAWINGS

[FIG. 1] FIG. 1 is a graph showing an effect of differences of concanavalin A concentration in a culture medium on the proliferation of the aβ-type T cells.
[FIG. 2] FIG. 2 is a graph showing an effect of the presence or absence of concanavalin A in a culture medium on the expression of interleukin-2 receptor on the surface of the canine peripheral lymphocytes.
[FIG. 3] FIG. 3 is a graph showing an effect of differences of serum concentration in a culture medium on the proliferation of the aβ-type T cells.
[FIG. 4] FIG. 4 is a graph showing an effect of differences of interleukin-2 concentration in a culture medium on the proliferation of the aβ-type T cells.
[FIG. 5] FIG. 5 is a graph showing the result of the culture of the aβ-type T cells obtained from different dogs in the presence of the autologous serum of each dogs.
[FIG. 6] FIG. 6 is a graph showing the result of the culture of the aβ-type T cells obtained from different dogs in the presence of the fetal bovine serum.
[FIG. 7] FIG. 7 is a graph showing the result of the culture of the aβ-type T cells obtained from different cats in the presence of the autologous serum of each cats or the fetal bovine serum.
[FIG. 8] FIG. 8 shows histograms of flowcytemetry measured before and after proliferation of the cells.
[FIG. 9] FIG. 9 is a graph showing the result of cytotoxicity of dog peripheral lymphocytes cultured in a culture medium supplemented with concanavalin A and interleukin-2 against human melanoma cells.
[FIG. 10] FIG. 10 is a photograph of electrophoresis showing the effect of the culture of dog peripheral lymphocytes in a culture medium supplemented with concanavalin A and interleukin-2 on mRNA transcription activity of granzyme B.
[FIG. 11] FIG. 11 is a graph showing the cytotoxicity of the peripheral lymphocytes against the human melanoma cells, in which the peripheral lymphocytes were obtained from the following steps of culturing dog peripheral lymphocytes in a culture medium supplemented with concanavalin A and interleukin-2, transferring the cultured dog peripheral lymphocytes to the dog from which the lymphocytes were originated, and obtaining the peripheral lymphocytes from the transferred dog.
[FIG. 12] FIG. 12 shows photographs showing the changes of an affected site of the dog suffering from a cancer, which underwent the treatment method of the present invention. In this figure, FIG. 12(a) is a photograph of the affected site before the treatment, and FIG. 12(b) is a photograph of the affected site after the treatment.
[FIG. 13] FIG. 13 shows photographs showing the changes of an affected site of the dog suffering from canine demodicosis (demodectic acariasis), which underwent the treatment method of the present invention. In this figure, FIG. 13(a) is a photograph of the affected site before the treatment, and FIG. 13(b) is a photograph of the affected site after the treatment.
[FIG. 14] FIG. 14 shows photographs showing the changes of another affected sites of the same dog as that of FIG. 13. In this figure, FIG. 14(a) is a photograph of the affected site before the treatment, and FIG. 13(b) is a photograph of the affected site after the treatment.

### EXPLANATION OF THE REFERENCE NUMERALS

1: tumor
2, and 3: affected sites

### BEST MODE FOR CARRYING OUT THE INVENTION

### 1. Method for proliferating LAK cells

A method for proliferating LAK cells of the present invention comprises the following step of (1) a proliferating/activating step by culturing cells in a start specimen in a culture medium supplemented with at least a plant lectin and a growth factor having interleukin-2-like activity and proliferating and activating the cells as set out in the claims. The respective steps of the present invention are hereinafter illustrated in detail.

### (1) Harvesting step

This step aims at collecting a start specimen containing aβ-type T cells from an animal.

### (a) Animal

The term "animal" refers to a dog or a cat, and the term "non-human animal" refers to a mammalian animal other than a human.

### (b) Start specimen

A bodily fluid or a tissue containing aβ-type T cells or progenitor cells thereof can be used as "a start specimen" for use in proliferation. Specifically, an example of a start specimen includes peripheral blood, umbilical cord blood, bone marrow, lymphatic tissue, epithelium, thymus, liver, spleen, cancerous tissue, infected tissue, lymph node tissue, embryonic tissue, or fractions thereof. Especially, fractions of peripheral blood are preferably used as a start specimen, considering a high density of aβ-type T cells and easiness of obtaining the same.

### (c) Harvest

Any known methods can be used to harvest a start specimen. Specifically, for example, in the case where a start specimen is bodily fluid, the start specimen can be harvested by layering a bodily fluid collected from a subject using an injection syringe over a solution having an appropriate specific gravity and sorting out appropriate fractions through the density gradient centrifugation method. Note that, in the case where a start specimen is enriched with red blood cells having specific gravity similar to that of aβ-type T cells, specifically in the case where a peripheral blood is used as a start specimen, a concentration of aβ-type T cells in the sample can be more increased by preliminarily disrupting red blood cells using a buffer solution such as NH₄Cl buffer solution. Further, in the case where a peripheral blood is used as a start specimen, heparin, citric acid and so on can be added to the start specimen to prevent from clotting.

Further, in the case where a tissue is used as a start specimen, a start specimen can be harvested by the following steps of: treating with trypsinization a piece of tissue excised from a subject by surgical operation to digest intercellular adhesion molecules, dispersing thus obtained cells in a culture medium to prepare a cell suspension, layering the cell suspension over a solution having an appropriate specific gravity, and sorting out appropriate fractions through the density gradient centrifugation method.

### (d) Others

The aβ-type T cells can be efficiently and dominantly proliferated by a known method of harvesting a start specimen by preliminarily enriching with aβ-type T cells using a centrifuge method. Examples of known methods include a method for enriching only aβ-type T cells by applying a cell suspension to an antibody-immobilized affinity chromatography or a method for eliminating only γd-type T cells from a cell suspension.

### (2) Proliferating/activating step

The "proliferating/activating step" is a step of proliferating/activating cells in thus harvested start specimen in a culture medium supplemented with at least a plant lectin and a growth factor having interleukin-2-like activity.

In the context of the present invention, the term "proliferating" means culturing a cell in vitro. Further, the term "culturing" means placing a cell in a culture medium supplemented with nutrient sources and so on, which are necessary for the cells to survive, and allowing the cells to grow in number. Moreover, the term "activating" means regulating a function of the cells such that functions of the cells is activated by stimulating the cells; more specifically, the term "activating" means inducing or improving an antitumor activity, an anti-immunological disease activity, or an anti-infectious diseases activity of the cells.

### (a) Plant lectin

The term "a plant lectin" means a glyco-binding protein derived from a plant body; specifically, the "plant lectin" includes, but not limited to, concanavalin A, phytohemagglutinin, porkweed mitogen, and so on. Considering the cost and availability of the lectin, concanavalin A is preferably used as a plant lectin. Further, a concentration of concanavalin A in the culture medium preferably ranges from 1 µg/ml to 15 µg/ml, more preferably ranges from 5 µg/ml to 10 µg/ml.

### (b) Growth factor having interleukin-2-like activity

The wording "a growth factor having interleukin-2-like activity" means any compounds having a similar ability to that of interleukin-2 to proliferate aβ-type T cells. Specifically, the "growth factor having interleukin-2-like activity" includes, but not limited to, interleukin-2 itself and a functional equivalent, such as a modified interleukin-2 with amino acid substitution(s), a partially digested interleukin-2, an interleukin-2 mimic, and so on. Also, as far as a compound satisfies the conditions described above, there is no need to limit the origin of the growth factors such as interleukin-2 and so on. For example, in the present invention, it is possible to use the growth factor derived from a human, a dog, a cat, a domestic animal, and so on. Among these, human interleukin-2 is preferably used as the growth factor due to its availability. A concentration of the growth factor having interleukin-2-like activity preferably ranges from 1 U/ml to 1000 U/ml, more preferably, ranges from 500 U/ml to 750 U/ml.

### (c) Proliferation and activation

### (c1) Culture medium

Any known culture media (without any limitation) such as a commercially available culture medium (such as, but not limited to, RPMI 1640 culture medium available from Nissui Pharmaceutical Co, Ltd.) can be used as a culture medium in the present invention, provided that the culture medium is supplemented with a plant lectin and a growth factor having interleukin-2-like activity. A serum free culture medium may be used in the art. However, to encourage the cell proliferation, a serum-containing culture medium is preferably used as a culture medium. Any commercially available serum, such as fetal bovine serum, may be used as serum. A serum which is homogeneously derived from an animal species from which a start specimen is harvested or its autologous serum may also be used. In the case where serum is added to the culture medium, the additive amount preferably ranges from 1 weight % to 20 weight % of the amount of the culture medium, and, more preferably, ranges from 2 weight % to 5 weight % of the amount of the culture medium.

### (c2) Culturing method

Cell culture may be conducted using a general culture method for culturing an animal cell, i.e., placing a culture vessel containing a culture medium in the CO₂ incubator. Any known culture vessels, including, as examples, a cell culture plate, a cell culture dish, a cell culture flask, a roller bottle,a gas-permeable culture bag and so on, may be used as a culture vessel.

A concentration of CO₂ in the CO₂ incubator preferably ranges 1-10%, and, more preferably, ranges 3-7%. Temperature in the CO₂ incubator preferably ranges 30-40°C, and, more preferably, ranges 35-38°C.

There are no need to limit the culture period, similar to the case of the usual animal cell culture. For proliferation and activation of cells, the culture period preferably ranges about 2 days-20 days, and, more preferably, ranges about 7 days -14 days. It is possible, during the culture period, to microscopically observe the state of the cells to count the number of the cells, to add a fresh culture medium or specific constituents thereof to the culture medium depending on the state of the cultured cells, and, in some cases, to change the culture medium or to passage the proliferated cells into a fresh culture medium contained in another culture vessel.

### 2. Cell suspension and cellular formulation

The cell suspension where the aβ-type T cells in a start specimen were proliferated or activated (enriched) using the method for proliferating LAK cells described in section 1 above may then be used in various types of experimentation as a cell suspension. After eliminating the culture medium, and a plant lectin and a growth factor having interleukin-2-like activity contained therein from the cell suspension (an eliminating step), the collected cells are resuspended in an appropriate solution at an appropriate cell density (a producing step) to prepare a cellular formulation. The eliminating step and the producing step are described below in detail.

Thus obtained cellular formulation contains the cells which have been proliferated by a method for proliferating LAK cells of the present invention. As far as the cellular formulation may be used for treating a disease, there is no need to limit a shape, a start specimen, an animal species from which a start specimen is harvested, a type of solution for use in re-suspension, and so on.

### (3) Eliminating step

After completion of the proliferating/activating step, to prevent an unwanted adverse immunoreaction potentially generated by the culture medium or the plant lectin, any known methods can be used to eliminate the culture medium or the plant lectin by collecting only the cells contained in the culture medium and washing out the culture medium adhered on the surface of the cells.

Specifically, a method for eliminating the culture medium or the plant lectin includes a method comprising steps of centrifuging the culture vessel to precipitate the cells, discarding the culture medium by decantation and re-suspending the cells in the buffering solution or a method using spin columns. The method for eliminating the culture medium or the plant lectin also includes a method using magnetic beads on which an antibody against aβ-type T cells is immobilized or affinity chromatographic technique, a method for isolating only the cells using a combination of a fluorescence antibody against aβ-type T cells and flow cytometric technique.

### (4) Producing step

After eliminating the culture medium, any known methods, such as a method comprising adding an appropriate solution to the culture flask containing the cells and suspending the cells by vortex mixing, can be used for suspending the collected cells in an appropriate solution at an appropriate cell density.

Any known solutions can be used as a solution for suspending the cells, provided that the solutions can be used to maintain an ability of LAK cells and does not cause any adverse immunoreaction such as immunological rejection in a non-human animal to which a cellular formulation containing this solution is administered. Specifically, the solution may include, as examples, an ordinary infusion solution (such as an infusion solution containing albumin, vitamin, glucose and so on), or the culture medium containing an autologous serum. The cell density in the solution varies depending on a start specimen, an animal species from which the cells are harvested, a type of solution for use in re-suspension. In this invention, cells at a density ranging 1 x 10⁵ cells/ml-1 x 10⁷ cells/ml, more preferably, at a density ranging 1 x 10⁶ cells/ml-6 x 10⁶ cells/ml, are preferably used for producing a cellular formulation.

### 3. Treatment method

Also described herein is a method for treating a non-human animal suffering from cancers or tumors, immunodeficiency diseases or infectious diseases by administering an effective amount of a cellular formulation, especially an effective amount of the cellular formulation originated from the start specimen of the non-human animal, to a non-human animal from which a start specimen is harvested (i.e., an administrating step). Methods of treatment per se are not claimed. The effective amount means a total amount of the cellular formulation necessary for a desired outcome (such as complete cure of a disease) and can be defined based on, for example, the number of the cells to be administered in a single dose, the number of doses a day, or the dosing days per one month.

### (5) Administrating step

### (a) Details of administration

Administration of the cells may be achieved by any known methods, provided that a known method can deliver a cellular formulation inside a body of a non-human animal, especially within a blood vessel, via infusion or injection. Infusion is preferably used to deliver a large number of LAK cells into the body of a non-human animal.

The applied dose for use in infusion varies depending on a subject animal species, a type of diseases to be treated, and so on. The cells are preferably administered at a dose of 1 x 10⁶ cells-5 x 10⁷ cells per 1 kg of body weight, more preferably at a dose of 5 x 10⁶ cells-1 x 10⁷ cells per 1 kg of body weight. The frequency of administration of the cells also varies depending on a subject animal species, a type of diseases to be treated. In the present invention, administration of the cells is preferably achieved by administering the cells at frequency of once/10 days-once/14 days or twice/month-three times/month, and, more preferably, at frequency of once/7 days-once/8 days or four times/month-five times/month.

### (b) Extent of diseases to be treated

Cancers or tumors to be treated by this treatment method may include, but not limited to, cancers or tumors such as pulmonary cancer, gastric cancer, hepatic cancer, pancreatic cancer, colon and rectal cancer, prostate cancer, breast cancer, uterine cancer, bladder cancer, leukemia, osteogenic sarcoma, cerebral tumor, and so on.

Immunodeficiency diseases to be treated by this treatment method may include, but not limited to, rheumatoid arthritis, systemic lupus erythematosus, Sjogren's syndrome, myasthenia gravis, malignant anemia, chronic thyroiditis (Hashimoto's disease), AIDS, and so on.

Infectious diseases to be treated by this treatment method may include, but not limited to, viral infectious diseases (such as canine distemper virus, rabies virus, parvovirus, feline immunodeficiency virus), bacterial infectious diseases, protozoan infectious diseases (such as filariasis and toxoplasmosis), mite infectious diseases (such as canine demodicosis (demodectic acariasis) and otodectic mange), and so on.

### 4. Kit for proliferation

Each components necessary for proliferation of the cells (such as a plant lectin, a growth factor having interleukin-2-like activity, culture medium, a cell culture vessel) used in the proliferation method of LAK cells of the invention can be purchased separately from various venders. Alternatively, it is possible to preliminarily combine these components to prepare a kit for proliferation, in order to relieve the skilled artisan of the bother of purchasing each component separately and to achieve proliferation of cells easier.

The invention of the kit for proliferation includes wide variety of kits such as a simple kit containing a necessary plant lectin and a growth factor having interleukin-2-like activity as set out in the claims, or a kit containing the culture medium, and other components such as other growth factors in addition to a necessary plant lectin and a growth factor. The kit for proliferation may be prepared by not only combining the components described above but also making any further modificaitons.

A growth factor having interleukin-2-like activity is dissolved in a solution, such as water, physiological saline, phosphate buffer solution, or the culture medium, and is frozen for preservation in small vessels each containing a single dose, which may be thawed upon use. The method for preserving the growth factor described above can eliminate the step of measurement of a growth factor having interleukin-2-like activity.

Further, the culture medium supplemented with a plant lectin and a growth factor having interleukin-2-like activity in a culture medium is frozen for preservation in small vessels, which may be thawed upon use. By such preservation method, it is possible to eliminate almost all procedures relating to proliferation or activation of cells.

Hereinafter, the invention is illustrated in detail by Examples. However, the following examples serve only to illustrate the invention described herein and are not intended to limit the scope of the invention.

### [EXAMPLES]

### Example 1

In this Example, an optimum proliferation condition for LAK cells was investigated using concanavalin A as a plant lectin and human interleukin-2 as a growth factor having interleukin-2-like activity.

### 1. Optimization of concanavalin A concentration

### (1) Harvesting of start specimen

The whole blood 30 ml was collected from the cervical vein of beagle (6-year-old), in an injection syringe (blood clotting was inhibited by heparin). Then, the collected whole blood was subjected to centrifugation, one volume of the collected peripheral blood pellet was combinded with 10 volume of any of solutions of (a) NH₄Cl buffering solution (NH₄Cl:0.83 g/100 ml of distillated water), (b) Tris base solution (20.6 g/1000 ml of distillated water, pH7.2), or (c) a filter-sterilized solution of the mixture of the solutions (a) and (b) at a ratio of 9:1, and then thus obtained suspension was placed in ice for 5 minutes (sometimes stirring), and red blood cells contained in the whole blood were destructed.

Three ml each of the specific gravity solution (for isolation of canine lymphocytes, Lympholyte available from Cedarlane) was equally poured into two centrifuge tubes and the destructed whole blood was slowly layered over the solution without disrupting the surface of solutions. The centrifuge tubes containing the cells were subjected to centrifugation at ambient temperature at 800 x g for 20 minutes to harvest fractions containing lymphocytes using an injection syringe or a sterile Pasteur pipette.

The cells were washed by suspending the harvested fractions in the culture medium to prepare cell suspensions, centrifuging thus obtained cell suspensions at 300 x g at ambient temperature for 5 minutes, and then discarding the supernatant by decantation. After washing the cells three times, the washed cells were suspended in 5 ml of the culture medium (the cell density: 4.76 x 10⁷ cells/ml). The culture medium RPMI 1640 (available from Nissui Pharmaceutical Co, Ltd., hereinafter simply referred to "the culture medium") was used as a culture medium.

### (2) Proliferation of cells

The cell suspensions in the centrifuge tubes were transferred to test tubes, to which the culture medium was added to adjust the cell density at 1 x 10⁶ cells/ml, then a final concentration of 2% or 10% of heat inactivated canine serum (harvested from a dog blood donor) inactivated at 56°C for 30 minutes or those of heat inactivated fetal bovine serum inactivated at 56°C for 30 minutes (Fetal Bovine Serum, available from JRH Biosciences) were added to the respective cell suspensions, and, finally, 2 ml each of cell suspensions were seeded in each well of two 6-well plates (2 x 10⁶ cells/well). Next, concanavalin A (Concanavalin A, available from Elastin Products) was added to the respective cell suspensions in each well at a final concentration of 1, 5, 10, or 15 µg/ml to prepare four set of different final concentration level of concanavalin A.

Then, the plates were placed in 37°C humidified 5% CO₂ incubator to start proliferation of cells. Increase and decrease of the number of the cells from the start of cellular proliferation to Day 14 were measured to calculate the average values for each concentration set of ConA. The number of the cells was calculated from the cell counts obtained by mixing 20 µl of a cell suspension and 20 µl of trypan blue solution and measuring the cell counts contained in 1 µl of the mixture using hemacytometers (Burker-Turk hemacytometers, available from ERMA) and microscope.

### (3) Experimental results

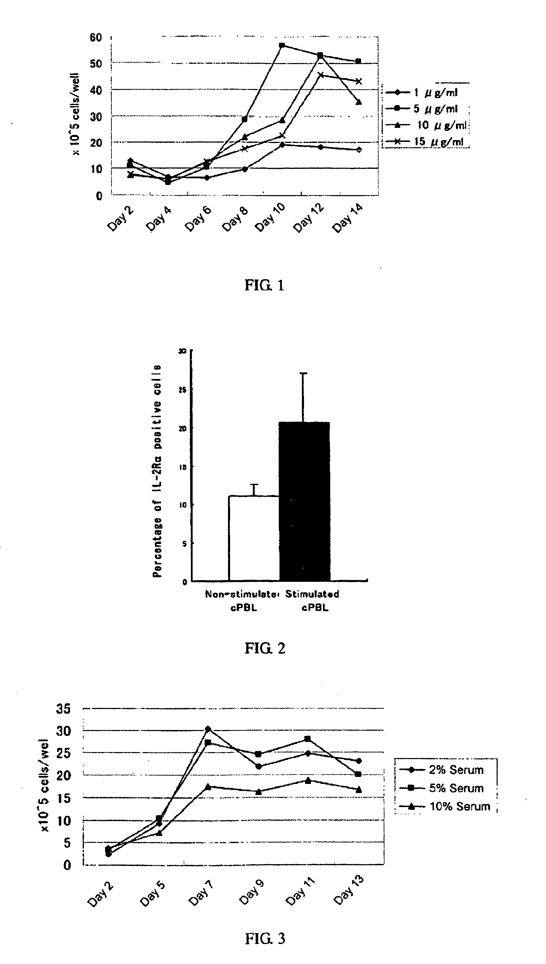

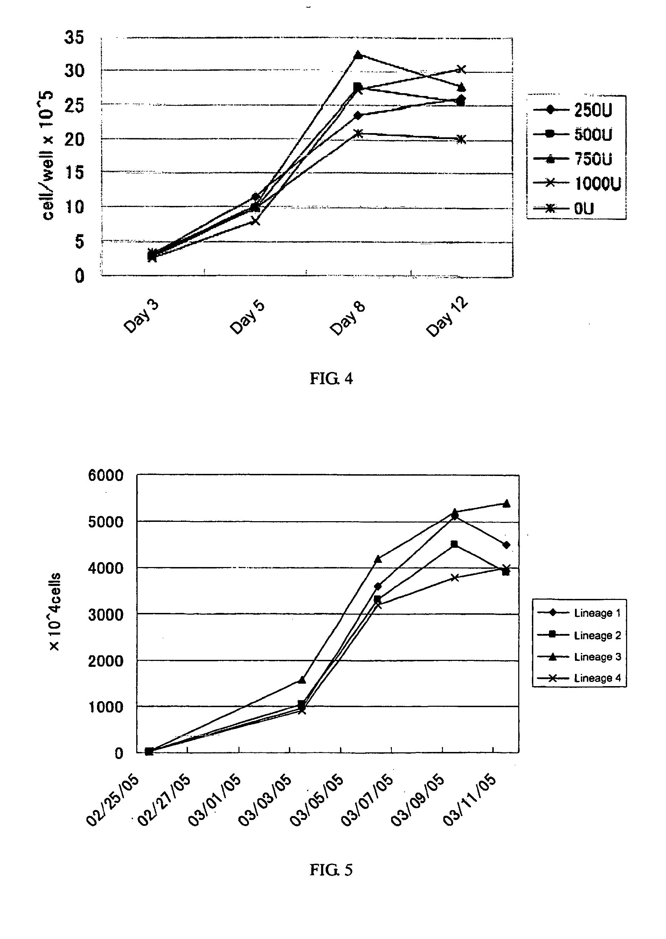

The changes in the number of the cells measured are shown in Table 1 and FIG. 1 prepared from the data of Table 1. As is clear from Table 1 and FIG. 1, 5 µg/ml of concanavalin A was found to be most appropriate concentration of ConA.

**[Table 1]**

| | 1 µg/ml | 5 µg/ml | 10 µg/ml | 15 µg/ml |
|---|---|---|---|---|
| Day 2 | 13 | 11 | 7.3 | 7.9 |
| Day 4 | 6.9 | 4.6 | 6.1 | 5.9 |
| Day 6 | 6.5 | 10 | 12 | 13 |
| Day 8 | 9.8 | 29 | 22 | 18 |
| Day 10 | 19 | 57 | 29 | 23 |
| Day 12 | 18 | 53 | 53 | 46 |
| Day 14 | 17 | 51 | 36 | 43 |
| x10^5 cells/well | | | | |

### 2. Change of lymphocytes by concanavalin A

The effect of concanavalin A on the functions of the lymphocytes, specifically the effect on the expression of interleukin-2 receptor a on the surface of the lymphocytes, was determined by flow cytometer.

### (1) Experimental procedure

Through the method similar to that of Example 1, three series (cases) of cell suspensions (5 ml each) were collected from 3 dogs (6-year-old) (the cell density: 2-4 x 10⁷ cells/ml). The culture medium was added to thus obtained each series of cell suspensions to adjust the cell density at 5 x 10⁵ cells/ml. Two ml each of cell suspensions were seeded in each well of 6-well plate (1 x 10⁶ cells/well) and, then, the cell suspensions were supplemented with a final concentration of 5 µg/ml of concanavalin A, a final concentration of 2% of canine serum (harvested from a dog blood donor), and a final concentration of 10%of fetal bovine serum and were cultured at 37°C for 5 days (Stimulated cPBL). The cell suspensions which were supplemented with canine serum and fetal bovine serum but were not with concanavalin A were used as a control (Non-stimulated cPBL).

After the culture, the cultured cells were collected by centrifugation and washed three times, from which 1 x 10⁶ cells were each transferred into test tubes, and 10 µl each of FITC-labeled mouse anti-human interleukin-2 receptor antibody was added to incubate at 4°C in the dark for 30 minutes. Mouse anti-human interleukin-2 receptor antibody purchased from American Research Products was used in this Example.

After incubation, the cells were washed with PBS three times and suspended in PBS, and, then, the ratio of the cells expressing interleukin-2 receptor a (IL-2Ra) on the surface of the cells was determined using flow cytometer (FACSCalibur, available from BECTON DICKINSON). The results are shown in FIG. 2. The data shown in this FIG.s are expressed as mean ± standard error.

### (2) Experimental results

As is clear from FIG. 2, the ratio of lymphocytes expressing the interleukin-2 receptor on the surface thereof was found to be high in the case where the cells were cultured in the culture medium supplemented with concanavalin A (Stimulated cPBL), compared to the case where the cells were cultured in the absense of concanavalin A (Non-stimulated cPBL) (statistically significant, P < 0.05). The data demonstrate that an addition of concanavalin A to the culture medium induces the expression of the interleukin-2 receptor.

### 3. Optimization of serum concentration

### (1) Experimental procedure

Through the method similar to that of the section 1 above, 5 ml of the cell suspension (the cell density: 1.38 x 10⁷ cells/ml) was prepared. The culture medium was added to the cell suspension to adjust the cell density at 5 x 10⁵ cells/ml, which was supplemented with final concentration of 5 µg/ml of concanavalin A, and then 1 ml each of the cell suspensions was seeded in each well of 6-well plate (5 x 10⁶ cells/well). Then, a final concentration of 2%, 5%, or 10% of the heat inactivated canine serum (harvested from a dog blood donor) inactivated at 56°C for 30 minutes was added to the cell suspensions in each wells to prepare 3 sets of cell suspensions containing varying final serum concentrations.

Then, the plate was placed in 37°C humidified 5% CO₂ incubator to start proliferation of cells. Increase and decrease of the number of the cells from the start of cellular proliferation to Day 13 were measured in the similar way to that used in the section 1 above.

### (2) Experimental results

The changes in the number of the cells measured are shown in Table 2 and FIG. 3 prepared from the data of Table 2. As is clear from Table 2 and FIG. 3, the concentration of 5% of serum was found to be most appropriate concentration of the serum.

**[Table 2]**

| | 2% serum | 5% serum | 10% serum |
|---|---|---|---|
| Day 2 | 2.3 | 3.4 | 3.8 |
| Day 5 | 9.3 | 10 | 7.2 |
| Day 7 | 31 | 27 | 18 |
| Day 9 | 22 | 25 | 16 |
| Day 11 | 25 | 28 | 19 |
| Day 13 | 23 | 20 | 17 |
| x10^5 cells/well | | | |

### 4. Optimization of interleukin-2 concentration

### (1) Experimental procedure

Through the method similar to that of the section 1 above, 5 ml of the cell suspension (the cell density: 1.68 x 10⁷ cells/ml) was prepared. The culture medium was added to the cell suspension to adjust the cell density at 5 x 10⁵ cells/ml, which was supplemented with a final concentration of 5 µg/ml of concanavalin A, a final concentration of 2% of canine serum (harvested from a dog blood donor), and a final concentration of 10% of fetal bovine serum, and 1 ml each of the cell suspensions (5 x 10⁵ cells/ml) was seeded in each well of 24-well plate. Then, a final concentration of 250 U, 500 U, 750 U, 1000 U, or 0 U/ml of interleukin-2 was added to the cell suspensions in each well to prepare 5 sets of cell suspensions containing varying final interleukin-2 concentration.

Then, the plate was placed in 37°C humidified 5% CO₂ incubator to start proliferation of cells. Further, at Day 5 from the start of cellular proliferation, a final concentration of 500 U of interleukin-2 and a final concentration of 5 µg/ml concanavalin A were added to the cell culture. Increase and decrease of the number of the cells from the start of cellular proliferation to Day 12 were measured in the similar way to that used in the section 1 above.

### (2) Experimental results

The changes in the number of the cells measured are shown in Table 3 and FIG. 4 prepared from the data of Table 3. As is clear from Table 3 and FIG. 4, the concentration of 750 U/ml of interleukin-2 was found to be most appropriate concentration of interleukin-2.

**[Table 3]**

| | 250 U | 500 U | 750 U | 1000 U | 0 U |
|---|---|---|---|---|---|
| Day 3 | 3.0 | 2.7 | 2.9 | 2.6 | 3.2 |
| Day 5 | 12 | 9.8 | 10 | 7.9 | 9.9 |
| Day 8 | 24 | 28 | 33 | 27 | 21 |
| Day 12 | 26 | 25 | 28 | 30 | 20 |
| x10^5 cells/well | | | | | |

### Example 2

The effects of types of serum used in the culture medium on the proliferation of LAK cells were determined using autologous serum, fetal bovine serum, or feline serum. Specifically, the effects were determined by the following procedures.

### 1. Use of autologous serum

### (1) Experimental procedure

Through the method similar to that of Example 1, four series of cell suspensions (1 ml each) were collected from 4 dogs (Miniature Pinscher, 2-year-old; mixed-breed, 1-year-old; Pomeranian, 10-year-old; mixed-breed, 3-year-old) (the cell density: 1.5 x 10⁶ cells/ml). The culture medium was added to thus obtained each series of the cell suspensions to adjust the cell density at 1 x 10⁵ cells/ml, and 3 ml each of the cell suspensions were poured into each well of 6-well plate (3 x 10⁵ cells/well), which was supplemented with a final concentration of 5 µg/ml of concanavalin A, a final concentration of 5% of autologous serum, and a final concentration of 750 U/ml of interleukin-2. The number of the cells was calculated at the start of cellular proliferation using hemacytometers, as is described in Example 1. The autologous serum used in this Example was prepared by placing the peripheral blood inside the clean bench overnight to coagulate, centrifuging the coagulated blood (at ambient temperature, at 1,500 x g for 10 minutes) to collect the supernatant, and treating the supernatant at 56°C for 30 minutes for heat inactivation.

Then, the 6-well plate was placed in 37°C humidified 5% CO₂ incubator to start proliferation of cells. Increase and decrease of the number of the cells from the start of cellular proliferation to Day 15 were measured using hemacytometers.

### (2) Experimental results

The changes in the number of the cells measured are shown in Table 4 and FIG. 5 prepared from the data of Table 4. As is clear from Table 4 and FIG. 5, the number of the cells upon termination of the proliferation varied by the series of cell suspensions and ranged 3.9-5.4 x 10⁷ cells/ml.

**[Table 4]**

| | Lineage 1 | Lineage 2 | Lineage 3 | Lineage 4 |
|---|---|---|---|---|
| Feb. 25 | 30 | 30 | 30 | 30 |
| Mar. 3 | 960 | 1050 | 1578 | 900 |
| Mar. 6 | 3600 | 3300 | 4200 | 3180 |
| Mar. 9 | 5130 | 4500 | 5220 | 3780 |
| Mar. 11 | 4500 | 3900 | 5400 | 4000 |
| x10^4 cells/well | | | | |

### 2. Use of fetal bovine serum

### (1) Experimental procedure

Through the method similar to that of Example 1, four series of cell suspensions (1 ml each) were collected from 4 dogs (Miniature Pinscher, 2-year-old; mixed-breed, 1-year-old; Pomeranian, 10-year-old; mixed-breed, 3-year-old) (the cell density: 1.5 x 10⁶ cells/ml). The culture medium was added to thus obtained each series of the cell suspension to adjust the cell density at 1 x 10⁵ cells/ml, and 3 ml each of the cell suspensions were poured into each well of 6-well plate (3 x 10⁵ cells/well), which were supplemented with a final concentration of 5 µg/ml of concanavalin A, a final concentration of 5% of fetal bovine serum (Fetal Bovine Serum, available from JRH Biosciences), and a final concentration of 750 U/ml of interleukin-2. The number of the cells was calculated at the start of cellular proliferation using hemacytometers.

### (2) Experimental results

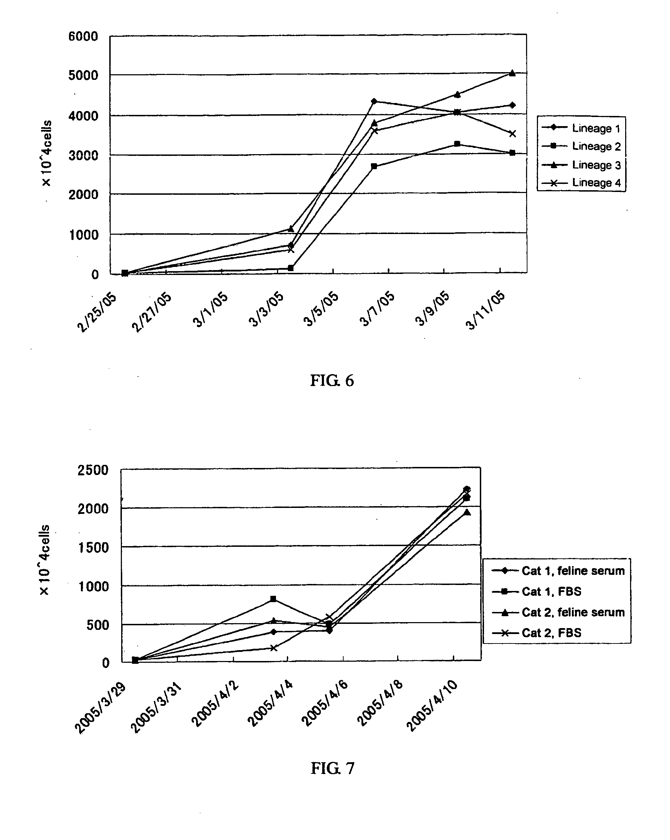

The changes in the number of the cells measured are shown in Table 5 and FIG. 6 prepared from the data of Table 5. As is clear from Table 5 and FIG. 6, the number of the cells upon termination of the proliferation varied by the series of cell suspensions and ranged 3.0-5.0 x10⁷ cells/ml.

**[Table 5]**

| | Lineage 1 | Lineage 2 | Lineage 3 | Lineage 4 |
|---|---|---|---|---|
| Feb. 25 | 30 | 30 | 30 | 30 |
| Mar. 3 | 720 | 120 | 1116 | 600 |
| Mar. 6 | 4320 | 2700 | 3780 | 3600 |
| Mar. 9 | 4050 | 3240 | 4500 | 4050 |
| Mar.11 | 4200 | 3000 | 5000 | 3500 |
| x10^4 cells/well | | | | |

### 3. Use of feline autologous serum

### (1) Experimental procedure

Through the method similar to that of Example 1, two series of cell suspensions (1 ml each) were collected from peripheral blood of 2 cats (mixed-breed, 2-year-old) (the cell density: 1.5 x 10⁶ cells/ml). The culture medium was added to thus obtained each series of the cell suspensions to adjust the cell density at 1 x 10⁵ cells/ml, and 3 ml each of the cell suspensions were poured into each well of two 6-well plates (3 x 10⁵ cells/well), which were supplemented with a final concentration of 5 µg/ml of concanavalin A, a final concentration of 5% of serum, and a final concentration of 750 U/ml of interleukin-2. In this example, fetal bovine serum was used in one series, while feline autologous serum was used in another series. The number of the cells was calculated at the start of cellular proliferation using hemacytometers. The feline autologous serum used in this Example was prepared by placing the peripheral blood inside the clean bench overnight to coagulate, centrifuging the coagulated blood (at ambient temperature, at 1,500 x g for 10 minutes) to collect the supernatant.

Then, the plates were placed in 37°C humidified 5% CO₂ incubator to start proliferation of cells. Increase and decrease of the number of the cells from the start of cellular proliferation to Day 15 were measured using hemacytometers.

### (2) Experimental results

The changes in the number of the cells measured are shown in Table 6 and FIG. 7 prepared from the data of Table 6. As is clear from Table 6 and FIG. 7, in the case of feline peripheral blood, the number of the cells upon termination of the proliferation less varied by the series of cell suspensions than that of canine peripheral blood and ranged 1.9-2.2 x 10⁷ cells/ml.

**[Table 6]**

| | Cat 1, feline serum | Cat 1, FBS | Cat 2, feline serum | Cat 2, FBS |
|---|---|---|---|---|
| Mar. 29 | 30 | 30 | 30 | 30 |
| Apr. 3 | 390 | 810 | 540 | 180 |
| Apr. 5 | 396 | 492 | 450 | 582 |
| Apr. 10 | 2220 | 2100 | 1920 | 2160 |
| x10^4 cells/well | | | | |

Comparing the result obtained in the Section 1 and the Section 2, it was found that there is no significant difference among the lineages in proliferation of canine aβ-type T cells between the aβ-type T cells cultured in the presence of canine autologous serum or those cultured in the presence of fetal bovine serum. Further, from the result obtained in the Section 3 which compares the case where the feline aβ-type T cells were cultured in the presence of feline autologous serum with the case where the cells were cultured in the presence of fetal bovine serum, it was also found that there is no significant difference in proliferation of feline aβ-type T cells between the experimental groups. As a result, it was found that proliferation of canine aβ-type T cells or feline aβ-type T cells is not practically dependent on the origin of the serum added to the culture medium.

### Example 3

In this Example, the changes in a cellular profile before and after proliferation were determined using a flow cytometer. Specifically, relating to CD8⁺ cells (which are aβ-type T cells and associated with cell-mediated immunity) and CD4⁺ cells (which are associated with antibody-mediated immunity), both of which were proliferated by a method for proliferation of the invention, the number of CD8⁺ cells and CD4⁺ cells and the ratio thereof were determined by the following procedure.

### (1) Experimental procedure

Through the method similar to that of Example 1, three series (cases) of cell suspensions (5 ml each) were collected from 3 dogs (6-year-old) (the cell density: 2-4 x 10⁷ cells/ml). The culture medium was added to thus obtained each series of cell suspensions to adjust the cell density at 1 x 10⁶ cells/ml. Two ml each of cell suspensions were seeded in each well of 6-well plate (2 x 10⁶ cells/well) and, then, the cell suspensions were supplemented with a final concentration of 5 µg/ml of concanavalin A, a final concentration of 5% of canine serum (harvested from a dog blood donor), a final concentration of 10% of fetal bovine serum, and a final concentration of 750 U/ml of interleukin-2.

The cell suspensions containing 1 x 10⁶ cells each were transferred into test tubes, and 10 µl each of FITC-labeled anti-canine CD8⁺ antibody and anti-canine CD4⁺ antibody were added to incubate at 4°C in the dark for 30 minutes. After incubation, the cells were washed with PBS three times and suspended in PBS, and, then, the profile of the cells was determined using a flow cytometer (FACSCalibur, available from BECTON DICKINSON). The anti-canine CD8⁺ antibody and anti-canine CD4⁺ antibody purchased from UK-Serotec were used in this Example.

Then, the plate was placed in 37°C humidified 5% CO₂ incubator to proliferate the cells for 7 days and the profile of the cells was determined using the above described method.

### (2) Experimental results

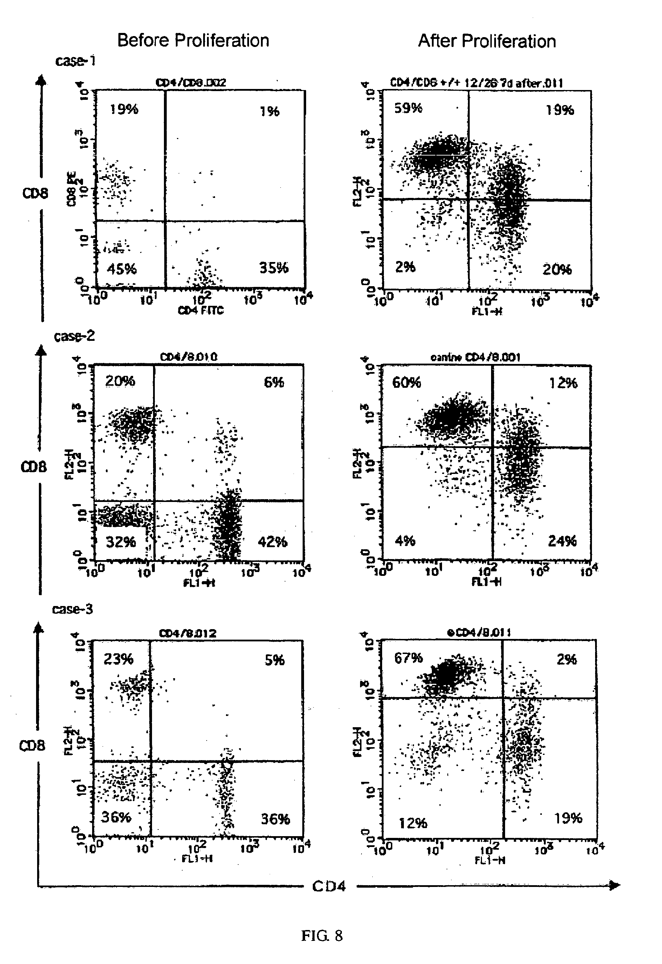

Histograms of the flow cytometer measured before the start of the cell culture and after the cell culture are shown in FIG. 8 and the values of the ratio between the number of the cells are shown in Table 7 based on the data shown in FIG. 8. In Table 7, "DN" indicates the percentage of the sum of CD8⁻ cells and CD4⁻ cells, "CD8 SP" indicates the percentage of CD8⁺ cells, "CD4 SP" indicates the percentage of CD4⁺ cells, "DP" indicates the percentage of CD8⁺ and CD4⁺ cells, respectively.

**[Table 7]**

| Before Proliferation | | | | | |
|---|---|---|---|---|---|
| | DN | CD4 SP | CD8 SP | DP | CD8 SP/CD4 SP |
| case-1 | 45 % | 35 % | 19 % | 1 % | 0.54 % |
| case-2 | 32 % | 42 % | 20 % | 6 % | 0.48 % |
| case-3 | 36 % | 36 % | 23 % | 5 % | 0.64 % |
| | | | | | |

| After Proliferation | | | | | |
|---|---|---|---|---|---|
| | DN | CD4 SP | CD8 SP | DP | CD8 SP/CD4 SP |
| case-1 | 2 % | 20 % | 59 % | 19 % | 2.95 % |
| case-2 | 4 % | 24 % | 60 % | 12 % | 2.5 % |
| case-3 | 12 % | 19 % | 67 % | 2 % | 3.53 % |

As is clear from FIG. 8 and Table 7, both the number and the percentage of CD8⁺ cells (CD8 SP) significantly increased in all cases. Further, it was found that the number and the percentage of CD8⁺ and CD4⁺ cells (DP) also increased. In contrast, though the number of CD4⁺ cells (CD4 SP) increased, the percentage of CD4⁺ cells (CD4 SP) decreased. From the result, aβ-type CD8⁺T cells are dominantly proliferated by the method of proliferating of the invention. Further, not only CD8⁺ cells (CD8 SP) but also CD8⁺ and CD4⁺ cells (DP) are proliferated, suggesting proliferation of any of MHC non-restricted lymphocytes, such as γdT cells and NK T cells. However, considering that the sample is derived from peripheral blood, there may be high possibility that NK T cells are proliferated.

### Example 4

In this Example, the cytotoxicity exerted by the proliferated cells against the tumor cells was examined. Specifically, after culturing lymphocytes in the culture medium supplemented with concanavalin A and interleukin-2 and co-culturing the cultured lymphocytes and human melanoma cells, a proliferation suppression effect on the lymphocytes exerted by the human melanoma cells and its mechanism were investigated.

### 1. Cytotoxicity of the proliferated cells

### (1) Experimental procedure

Through the method similar to that of Example 1, three series (cases) of cell suspensions (5 ml each) were collected from 3 dogs (6-year-old) (the cell density: 2-4 x 10⁷ cells/ml). The culture medium was added to thus obtained each series of cell suspensions to adjust the cell density at 5 x 10⁵ cells/ml. Two ml each of cell suspensions were seeded in each well of 6-well plate (1 x 10⁶ cells/well) and, then, the cell suspensions were supplemented with a final concentration of 5 µg/ml of concanavalin A, a final concentration of 2% of canine serum (prepared from a dog blood donor by the similar method to that of Example 2), a final concentration of 10% of fetal bovine serum, and a final concentration of 750 U/ml interleukin-2, which were cultured at 37°C for 7 days or 11 days.

Thus obtained lymphocytes as effector cells and human melanoma cells (MeWo cells) as target cells were co-cultured in the culture medium and the cytotoxicity exerted by the effector cells against the target cells was investigated. Specifically, the cytotoxicity was examined by the following method.

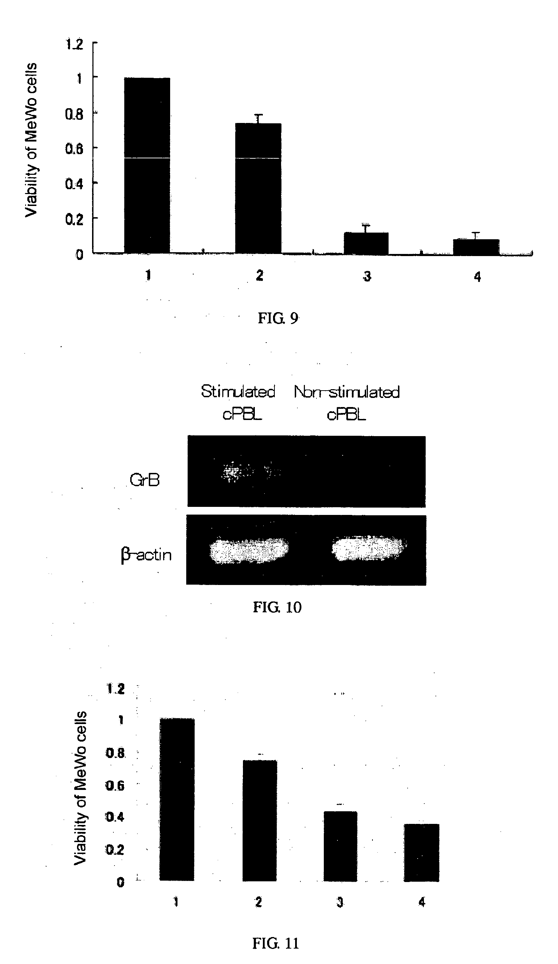

First, the effector cells and the target cells were seeded in each well of 24-well plates at an E:T ratio of 33 (i.e., 4.95 x 10⁶ effector cells/well) : 1 (i.e., 1.5 x 10⁵ target cells/well) and were cultured at 37°C for 16 hours. In this step, the culture medium supplemented with a final concentration of 2% of canine serum (prepared from a dog blood donor by the similar method to that of Example 2), and a final concentration of 10% of fetal bovine serum were used for the co-culture. Then, the number of the viable target cells (MeWo cells) were counted using hemacytometers, and the viability of the target cells was calculated based on the cell counts. The results were shown in FIG. 9.

The experimental result which is obtained from the culture of only MeWo cells and the experimental result which is obtained by co-culturing MeWo cells and non-stimulated canine peripheral lymphocytes were used as control results. In FIG. 9: the column 1 indicates a control experimental result which is obtained from the culture of only MeWo cells; the column 2 indicates a control experimental result which is obtained by co-culturing MeWo cells and non-stimulated canine peripheral lymphocytes; the column 3 indicates an experimental result which was obtained by co-culturing the MeWo cells with the stimulated canine peripheral lymphocytes which were cultured in the presence of concanavalin A and interleukin-2 for 7 days; and the column 4 indicates an experimental result which was obtained by co-culturing the MeWo cells with the stimulated canine peripheral lymphocytes which were cultured in the presence of concanavalin A and interleukin-2 for 11 days.

As is clear from FIG. 9, comparing with the data obtained without the co-culture (the column 1) and the data obtained by the co-culture with the non-stimulated canine peripheral lymphocytes (the column 2), it was found that the co-culture with the stimulated lymphocytes significantly decreased the viability of the target cells (i.e., MeWo cells) as shown in the column 3 and the column 4 (statistically significant, P < 0.05). This result suggests the applicability of using the stimulated lymphocytes in the treatment of cancers.

2. Effect of granzyme B (GrB) on transactivation of mRNA

In this section, the effect of the supplementation of concanavalin A and interleukin-2 to.the culture medium on the GrB mRNA transcription activity, indicative of the cytotoxicity, was determined. Specifically, the cytotoxicity was examined by the following method.

### (1) Experimental procedure

First, the stimulated canine peripheral lymphocytes which were cultured in the presence of concanavalin A and interleukin-2 for 7 days as described in the Section 1 above (Stimulated cPBL) were used to extract the total RNA using Trizol reagent (available from Life Technologies).

Then, TaKaRa RNA PCR Kit (available from TAKARA BIO) was used to synthesize cDNAs from the total RNA, and the synthesized cDNAs were used as templates to conduct PCR reaction (RT-PCR method). In this experimentation, PCR reaction conditions, synthesis of primers for amplifying the canine GrB gene, and synthesis of primers for amplifying the canine β-actin gene used as an internal standard were designed according to the descriptions of a publication of Ito H., et al. (Ito H., et al., Neuromuscul. Disord. 8, 95-110 (1998)).

Finally, the thus obtained PCR products were subjected to electrophoresis and were compared to the control experimentation. The results are shown in FIG. 10. The canine peripheral lymphocytes cultured in the culture medium for 4 days in the absence of concanavalin A and interleukin-2 (Non-stimulated cPBL) were used as a control.

### (2) Experimental results

As is clear from FIG. 10, it was found that an amount of GrB transcription more increased in the stimulated lymphocytes which were cultured in the culture medium supplemented with concanavalin A and interleukin-2 (Stimulated cPBL), compared with the control group (Non-stimulated cPBL). The result shown in FIG. 10 suggests that the cell culture, stimulating the lymphocytes by culturing the lymphocytes in the culture medium supplemented with concanavalin A and interleukin-2, increases an transcription level of the GrB mRNA and an expression level of the GrB gene and improves the cytotoxicity exerted by the stimulated lymphocytes.

### 3. Effect of transfer of lymphocytes on cytotoxicity

The lymphocytes stimulated using concanavalin A and interleukin-2 were transferred back to the subject individual from which the lymphocytes had been originally harvested, and the lymphocytes were again harvested again from the subject individual to measure the cytotoxicity of the lymphocytes. Then, effects of the transfer of the stimulated lymphocytes on the cytotoxicity of the lymphocytes of the subject individual to be administered were examined.

### (1) Experimental procedure

Through the method similar to that of Example 1, three series (cases) of canine peripheral lymphocytes were collected from 3 dogs (6-year-old). The canine peripheral lymphocytes were stimulated by culturing for 7 days as described in the Section 1 above.

The cells were washed by centrifuging the stimulated canine peripheral lymphocytes at 300 x g at ambient temperature for 5 minutes to form precipitate, then discarding the supernatant by decantation. After washing the cells three times, the washed cells were suspended in 5 ml of the physiological saline to prepare a cellular formulation. A cellular formulation was prepared by suspending the lymphocytes which were cultured in the absense of concanavalin A and interleukin-2 (non-stimulated canine peripheral lymphocytes) in the similar method.

The cellular formulation was transferred back to the subject individual from which the lymphocytes had been originally harvested, and the lymphocytes were again harvested again from the peripheral blood of the subject individual. Specifically, in the Group 1, the subject dog individual was introduced by the cellular formulation (1 x 10⁸ cells/dog) for 4 weeks in a row, and after two days from the final introduction of the cellular formulation, the lymphocytes were harvested from the peripheral blood. In the Group 2, the subject dog individual was introduced by the cellular formulation (1 x 10⁸ cells/dog) for 5 weeks in a row, and after two days from the final introduction of the cellular formulation, the lymphocytes were harvested from the peripheral blood.

Thus obtained lymphocytes as effector cells and human melanoma cells (MeWo cells) as target cells were co-cultured in the culture medium as described in the Section 1 above and the cytotoxicity exerted by the effector cells against the target cells was investigated. The results were shown in FIG. 11.

The experimental result which is obtained from the culture of only MeWo cells and the experimental result which is obtained by co-culturing MeWo cells and non-stimulated canine peripheral lymphocytes were used as control results. In FIG. 11: the column 1 indicates a control experimental result which is obtained from the culture of only MeWo cells; the column 2 indicates a control experimental result which is obtained by co-culturing MeWo cells and non-stimulated canine peripheral lymphocytes; the column 3 indicates an experimental result which was obtained by co-culturing the MeWo cells with the stimulated canine peripheral lymphocytes of the Group 1 (being introduced for 4 weeks in a row); and the column 4 indicates an experimental result which was obtained by co-culturing the MeWo cells with the stimulated canine peripheral lymphocytes of the Group 2 ((being introduced for 5 weeks in a row).

### (2) Experimental results

As is clear from FIG. 11, comparing with the data obtained without the co-culture (the column 1) and the data obtained by the co-culture with the non-stimulated canine peripheral lymphocytes (the column 2), it was found that the co-culture with the stimulated lymphocytes significantly decreased the viability of the target cells (i.e., MeWo cells) as shown in the column 3 and the column 4 (statistically significant, P < 0.05). This result suggests that the cytotoxicity of the lymphocytes is maintained even after introduction of the lymphocytes into the body of teh subject individual.

### Example 5

In this Example, a therapeutic effect exerted by the proliferated cells was investigated. Specifically, a cellular formulation containing the proliferated aβ-type T cells which were grown by the method for proliferation of the invention was administered to model animals suffering from cancers or canine demodicosis (demodectic acariasis) (cutaneous disorder) to determine the therapeutic effect.

### 1. Therapeutic effect on cancers

Through the method similar to that of Example 1, 1 ml of a cell suspension was collected from a dog bearing a tumor (histiocytoma) located in the neck (mixed-breed, 14-year-old, 17 kg of body weight) (the cell density: 1.5 x 10⁶ cells/ml). The thus obtained cell suspension was transferred to a cell culture bottle (25 cm²), to which 9 ml of the culture medium were added (i.e., 10 ml in total). The cell suspension were supplemented with a final concentration of 5 µg/ml of concanavalin A, a final concentration of 5% of autologous serum, and a final concentration of 750 U/ml of interleukin-2. An autologous serum used in this Example was prepared in the similar way to that of Example 2.

Then, the cell culture bottle was placed in 37°C humidified 5% CO₂ incubator to proliferate the cells for 10 days. The cells were washed by centrifuging the cell suspension at 300 x g at ambient temperature for 5 minutes to form precipitate, then discarding the supernatant of the culture medium by decantation, resuspending the pellet of the cells in the physiological saline, and centrifuging again to discard the supernatant. After washing the cells three times, the washed cells were suspended in 30 ml of the physiological saline for use as an infusion solution (physiological saline, available from Otsuka Pharmaceutical Co.,Ltd.) to prepare a cellular formulation, and were filled in transfusion bag. The cells were existed in the cellular formulation at a density of 1.67 x 10⁶ cells/ml.

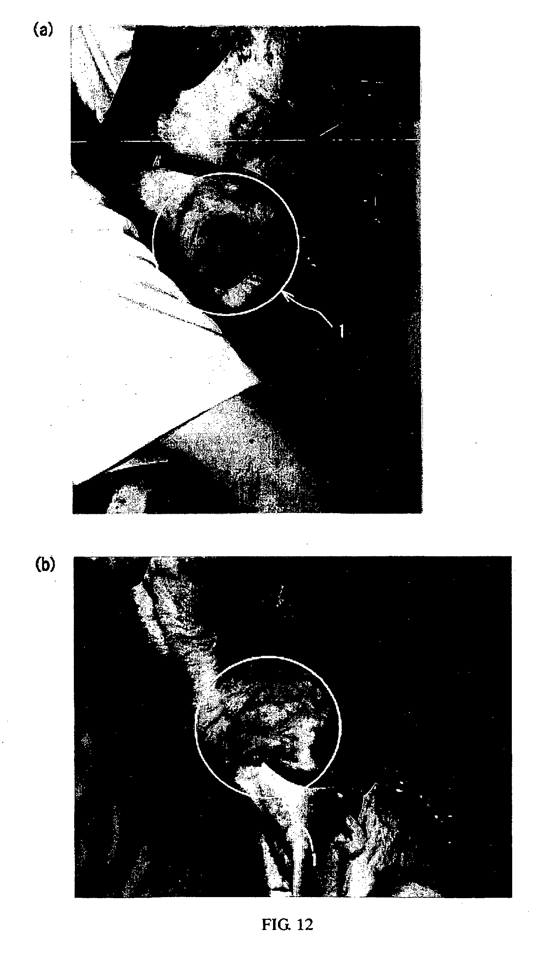

The cellular formulation was administered once to the dog bearing the tumor by infusion route. The results are shown in FIG. 12. FIG. 12(a) is a photograph of the tumor No. 1 before the treatment, and FIG. 12(b) is a photograph of the tumor No. 1 after the treatment. It was found from these photographs that the tumor 1 has been clearly degenerated, demonstrating that the treatment method of the invention is effective.

### 2. Effectiveness of therapy on the canine demodicosis (demodectic acariasis)

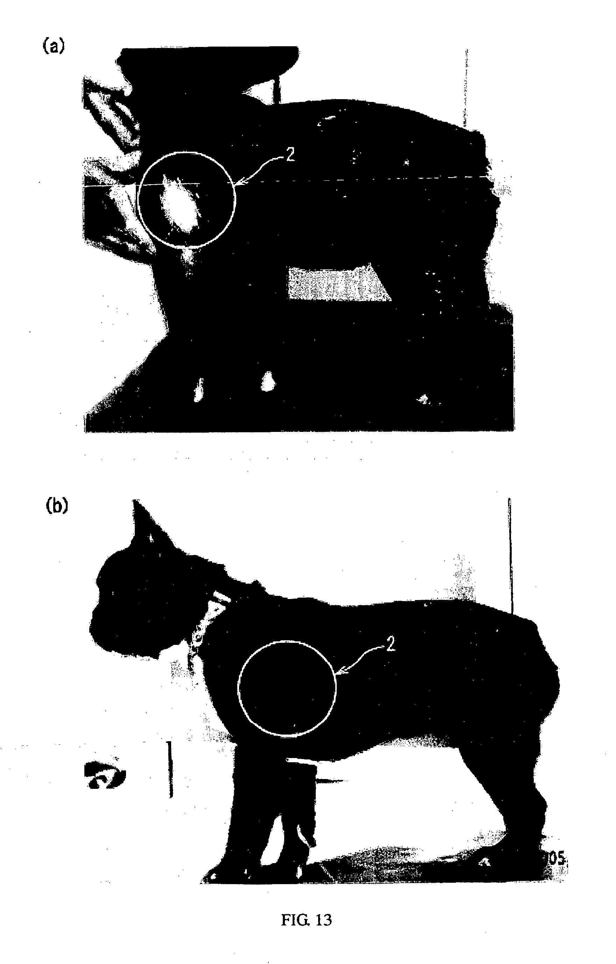

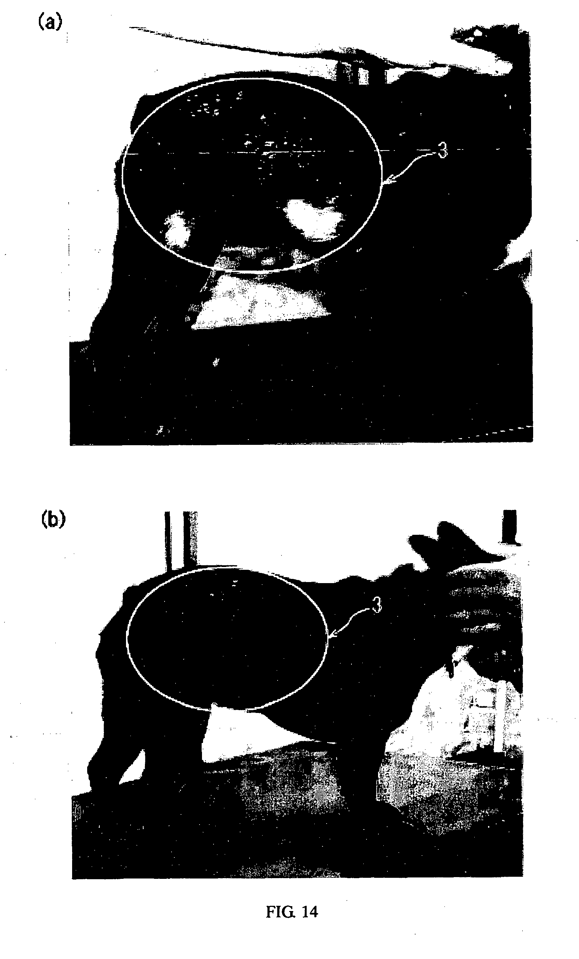

Through the method similar to that of Example 1, a cellular formulation was prepared from a dog suffering from canine demodicosis (demodectic acariasis) (French Bulldog, 2-year-old, 12 kg of body weight). The cells were existed in the cellular formulation at a density of 1.5 x 10⁶ cells/ml. The cellular formulation was administered once to the dog suffering from the canine demodicosis (demodectic acariasis) by infusion route. The results are shown in FIG. 13 and FIG. 14. FIG. 13 and FIG. 14 are photographs of two distinct affected sites of the single dog individual. FIG. 13(a) and FIG. 14(a) are photographs of the affected sites 2 and 3 before the treatment, respectively, and FIG. 13(b) and FIG. 14(b) photograph of the affected sites 2 and 3 after the treatment, respectively. These photographs demonstrate that hair grows at affected sites 2 and 3 after the treatment of the present invention. Therefore, the treatment method of the invention is effective.

### INDUSTRIAL APPLICABILITY

A method for proliferating LAK cells of the present invention can predominantly proliferated and activated aβ-type T cells demonstrating antigen specificity or MHC restriction. Since the thus obtained LAK cells contains relatively high percentage of aβ-type T cells having MHC restriction, the thus obtained LAK can be used for more effective activated autologous lymphocyte therapy, resulting in decreasing physical burden and economic burden of a patient to be treated suffering from various diseases (such as cancers, immunodeficiency diseases, and infectious diseases).

## Claims

1. A method for proliferating LAK cells, which comprises the following steps in number order:
(1) a proliferating/activating step by proliferating and activating the cells in a start specimen containing αβ-type T cells obtained from a dog or cat by culturing the cells in a culture medium supplemented with at least a plant lectin and a growth factor having interleukin-2-like activity, wherein the plant lectin is 1-15 µg/ml of concanavalin A and the growth factor is 500-750 U/ml of interleukin-2.

2. The method of Claim 1, wherein the start specimen containing αβ-type T cells is selected from at least one of the components consisting of peripheral blood, bone marrow, lymphatic tissue, epithelium, thymus, liver, spleen, cancerous tissue, infected tissue, lymph node tissue, embryonic tissue, and fractions thereof.

3. The method of Claim 2, wherein the start specimen is selected from at least one of the components consisting of peripheral blood, and fractions thereof.

4. The method of any one of Claims 1-3, further comprising the following steps in number order:
(2) an eliminating step by eliminating the culture medium and washing the cells after the proliferating/activating step; and
(3) a producing step by suspending the cells washed in an appropriate solution.

5. The method of any one of Claims 1-4, wherein the concentration of concanavalin A contained in the culture medium is 5 µg/ml.

6. The method of any one of Claims 1-4 and 5, wherein the concentration of interleukin-2 is 750 U/ml.

7. A kit for proliferating LAK cells obtained from a dog or cat individual comprising a growth medium containing at least a plant lectin and a growth factor having interleukin-2-like activity,
wherein the plant lectin is 5 µg/ml of concanavalin A and the growth factor is 500-750 U/ml of interleukin-2, the plant lectin is 1-15 µg/ml of concanavalin A and the growth factor is 750 U/ml of interleukin-2, or the plant lectin is 5 µg/ml of concanavalin A and the growth factor is 750 U/ml of interleukin-2.

8. The kit of Claim 7 which contains a cell culture vessel.

## Patentansprüche

1. Verfahren zur Proliferation von LAK-Zellen, das die folgenden Schritte in der Reihenfolge ihrer Nummerierung umfasst:
(1) einen Proliferations-/Aktivierungsschritt durch Proliferieren und Aktivieren der Zellen in einer Anfangsprobe, die T-Zellen des Typs αβ enthält, die von einem Hund oder einer Katze erhalten wurden, mittels Kultivierens der Zellen in einem Nährmedium, das mit zumindest einem pflanzlichen Lectin und einem Wachstumsfaktor mit Interleukin-2-artiger Aktivität ergänzt ist, worin das pflanzliche Lectin 1 bis 15 µg/ml Concanavalin A ist und der Wachstumsfaktor 500 bis 750 U/ml Interleukin-2 ist.

2. Verfahren nach Anspruch 1, worin die Anfangsprobe, die T-Zellen des Typs αβ enthält, aus zumindest einer der aus peripherem Blut, Knochenmark, Lymphgewebe, Epithel, Thymus, Leber, Milz, Krebsgewebe, infiziertem Gewebe, Lymphknotengewebe, Embryonengewebe und Fraktionen davon bestehenden Komponenten ausgewählt ist.

3. Verfahren nach Anspruch 2, worin die Anfangsprobe aus zumindest einer der aus peripherem Blut und Fraktionen davon bestehenden Komponenten ausgewählt ist.

4. Verfahren nach einem der Ansprüche 1 bis 3, das weiters die folgenden Schritte in der Reihenfolge ihrer Nummerierung umfasst:
(2) einen Eliminierungsschritt durch Eliminieren des Nährmediums und Waschen der Zellen nach dem Proliferations-/Aktivierungsschritt; und
(3) einen Herstellungsschritt durch Suspendieren der gewaschenen Zellen in einer geeigneten Lösung.

5. Verfahren nach einem der Ansprüche 1 bis 4, worin die Konzentration von in dem Nährmedium enthaltenem Concanavalin A 5 µg/ml beträgt.

6. Verfahren nach einem der Ansprüche 1 bis 4 und 5, worin die Konzentration von Interleukin-2 750 U/ml beträgt.

7. Set zur Proliferation von LAK-Zellen, die von einem Hund oder einer Katze stammen, das ein Wachstumsmedium umfasst, welches zumindest ein pflanzliches Lectin und einen Wachstumsfaktor mit Interleukin-2-artiger Aktivität umfasst,
worin das pflanzliche Lectin 5 µg/ml Concanavalin A und der Wachstumsfaktor 500 bis 750 U/ml Interleukin-2 ist, das pflanzliche Lectin 1 bis 15 µg/ml Concanavalin A und der Wachstumsfaktor 750 U/ml Interleukin-2 ist oder das pflanzliche Lectin 5 µg/ml Concanavalin A und der Wachstumsfaktor 750 U/ml Interleukin-2 ist.

8. Set nach Anspruch 7, das ein Zellkulturgefäß enthält.

## Revendications

1. Procédé de prolifération de cellules LAK, qui comprend les étapes suivantes en ordre de nombre :
(1) une étape de prolifération/activation par la prolifération et l'activation des cellules dans un spécimen de départ contenant des cellules T de type aß obtenues d'un chien ou d'un chat en cultivant les cellules dans un milieu de culture supplémenté avec au moins une lectine de plante et un facteur de croissance ayant une activité semblable à l'interleukine-2, où la lectine de plante est 1-15 µg/ml de concanavaline A, et le facteur de croissance est 500-750 U/ml d'interleukine-2.

2. Procédé selon la revendication 1, dans lequel le spécimen de départ contenant des cellules T de type aß est sélectionné parmi au moins un des composants consistant en sang périphérique, moelle osseuse, tissu lymphatique, épithélium, thymus, foie, rate, tissu cancéreux, tissu infecté, tissu du ganglion lymphatique, tissu embryonnaire et des fractions de ceux-ci.

3. Procédé selon la revendication 2, dans lequel le spécimen de départ est sélectionné parmi au moins un des composants consistant en sang périphérique et des fractions de celui-ci.

4. Procédé selon l'une quelconque des revendications 1 à 3, comprenant en outre les étapes suivantes selon l'ordre de nombre :
(2) une étape d'élimination consistant à éliminer le milieu de culture et à laver les cellules après l'étape de prolifération/activation ; et
(3) une étape de production en suspendant les cellules lavées dans une solution appropriée.

5. Procédé selon l'une quelconque des revendications 1 à 4, dans lequel la concentration de concanavaline A se trouvant dans le milieu de culture est de 5 µg/ml.

6. Procédé selon l'une quelconque des revendications 1 à 4 et 5, dans lequel la concentration d'interleukine-2 est de 750 U/ml.

7. Kit pour la prolifération de cellules LAK obtenues d'un chien ou d'un chat individuel comprenant un milieu de croissance contenant au moins une lectine de plante et un facteur de croissance ayant une activité semblable à l'interleukine-2,
où la lectine de plante est 5 µg/ml de concanavaline A, et le facteur de croissance est 500-750 U/ml d'interleukine-2, la lectine de plante est 1-15 µg/ml de concanavaline A, et le facteur de croissance est 750 U/ml d'interleukine-2, ou la lectine de plante est 5 µg/ml de concanavaline A, et le facteur de croissance est 750 U/ml d'interleukine-2.

8. Kit selon la revendication 7, qui contient un récipient de culture des cellules.
